# EUROPEAN PATENT APPLICATION

(11) **EP 3 290 009 A1**
(43) Date of publication of application: **07.03.2018**
(21) Application number: 17183646.3
(22) Date of filing: 27.07.2017
(51) Int. Cl.: A61F 5/055

(54) **NOVEL CERVICAL VERTEBRA TRACTOR**

(30) Priority: 29.08.2016 CN 201621045587 U
(71) Applicant: Hengshui Leamai Medical Instruments Co., Ltd., Jizhou District Hengshui City Hebei (CN)
(72) Inventor: Tian, Ying, Jizhou City, Hebei (CN)
(74) Representative: Cabinet Chaillot

(57) **Abstract**

The present utility model discloses a novel cervical vertebra tractor in which Velcro hook sides are attached respectively to left and right ends of a cervical collar support, Velcro loop sides are provided respectively at two ends of an inner side of a traction belt, and the Velcro loop sides and the Velcro hook sides are adhered to each other. A first cushion is provided at the middle portion of the inner side of the traction belt. Multiple vent holes are provided at the middle portion of the traction belt. A balloon is provided inside the traction belt. One end of an inflation tube is connected to the balloon, and the other end of the inflation tube is provided with a connector.

## Description

### FIELD OF THE INVENTION

The present utility model relates to the technical field of medical and health products, and particularly to a novel cervical vertebra tractor.

### BACKGROUND OF THE INVENTION

Therapies for cervical spondylosis include non-surgical treatments and surgical treatments. Among the non-surgical treatments, traction is a prevailing means of alleviating the symptoms in the treatment of cervical spondylosis. Effective traction can eliminate the muscle spasm in the neck, alleviate the pains, increase the intervertebral space and the intervertebral foramen, facilitate the stabilization of protruded nucleus pulposus and annulus fibrosis, alleviate and relieve the nerve root compression and stimulation, promote the absorption of nerve root edema, relieve the compression of the vertebral artery by the vertebra, promote the blood circulation, facilitate the regression of local congestion, swelling, and hyperplasia, relax the adhesive joint capsules, ameliorate and recover the Luschka joint, regulate the facet joint dislocation and spondylolisthesis, adjust and restore the destroyed internal and external balance of the cervical vertebra, and restore the normal functions of the cervical vertebra. A cervical vertebra tractor is a product for cervical spondylosis treatment designed on the basis of such a principle and which can be used conveniently by the patients at home.

At present, existing cervical vertebra tractors are designed to have a one-piece structure that cannot be adjusted at will according to the body sizes of the users. Moreover, the rear side of the traction belt, when inflated, cannot form a physiological curve angle, which does not conform to the principle of human cervical vertebra recovery and cannot achieve an effective traction of the cervical vertebra, thus being failed to meet the users' requirements.

### SUMMARY OF THE INVENTION

In view of the problems mentioned above, it is an object of the present utility model to provide a novel cervical vertebra tractor.

A novel cervical vertebra tractor according to the present utility model includes a cervical collar, a cervical collar support, a plastic rivet, Velcro hook sides, a traction belt, Velcro loop sides, a first cushion, vent holes, an inflation tube, a connector, a hand press ball, and an air stopping knob. The cervical collar is provided with the cervical collar support at a middle portion at a front end thereof, and the cervical collar support is fastened to the cervical collar through a plastic rivet. The Velcro hook sides are attached respectively to left and right ends of the cervical collar support, and the two Velcro hook sides are fastened respectively to two ends of an outer side of the cervical collar. The Velcro loop sides are provided respectively at two ends of an inner side of the traction belt. The Velcro loop sides at the two ends of the traction belt are adhered respectively to the Velcro hook sides at the two ends of the outer side of the cervical collar. The first cushion is provided at the middle portion of the inner side of the traction belt. Multiple vent holes are provided at the middle portion of the traction belt. A balloon is provided inside the traction belt. One end of the inflation tube is connected to the balloon and the other end of the inflation tube is provided with a connector. The other end of the inflation tube is rotatably connected to one end of the hand press ball through the connector. The air stopping knob is provided on the hand press ball.

Preferably, the cervical collar is a polymer cervical collar made of a soft spongy material.

The present utility model has the following beneficial effects. The cervical vertebra tractor of the present utility model is designed to have a separable structure in which the cervical collar and the traction belt are assembled through a Velcro before use. As such, the cervical vertebra tractor is adjustable to different body sizes of the users. By providing a cervical collar support on the cervical collar, not only can the supporting strength for the cervical vertebra be enhanced, but also the cervical vertebra can be prevented from deformation. By using both an air pump and a hand press ball for inflation, users can choose an inflation means at will depending on their usage habits. By providing a balloon in the traction belt, the traction belt can be inflated into a curved shape which conforms to the physiological curve of the human body, thereby achieving an effective traction.

### BRIEF DESCRIPTION OF THE DRAWINGS

For ease of description, the present utility model shall be described in detail with reference to the following embodiments and accompanying drawings, in which:
FIG.1 is a schematic view showing the structure of the present utility model;
FIG.2 is a rear view of the present utility model;
FIG.3 is a schematic view showing the structure of a traction belt according to the present utility model;
FIG.4 is a schematic view showing the inner structure of a traction belt according to the present utility model;
FIG. 5 is a schematic view showing the structure of a cervical collar according to the present utility model;
FIG. 6 is a schematic view showing the structure of a hand press ball according to the present utility model;
FIG. 7 is a schematic view showing the structure of a second embodiment;
FIG. 8 is a rear view of the second embodiment; and
FIG. 9 is a schematic view showing the structure of an air pump according to the second embodiment.

### Reference numbers:

1 cervical collar; 1-1 second cushion; 2 cervical collar support; 3 plastic rivet; 4 Velcro hook side; 5 traction belt; 5-1 plastic support; 6 Velcro loop side; 7 first cushion; 8 vent hole; 9 inflation tube; 10 connector; 11 hand press ball; 12 air stopping knob; 13 air pump; 13-1 connection port; 13-2 extension tube; 13-3 connector locking means.

### DETAILED DESCRIPTION OF THE INVENTION

### First Embodiment

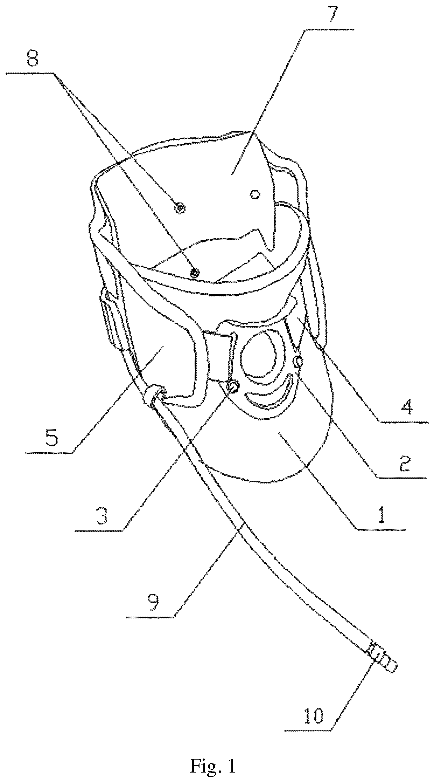

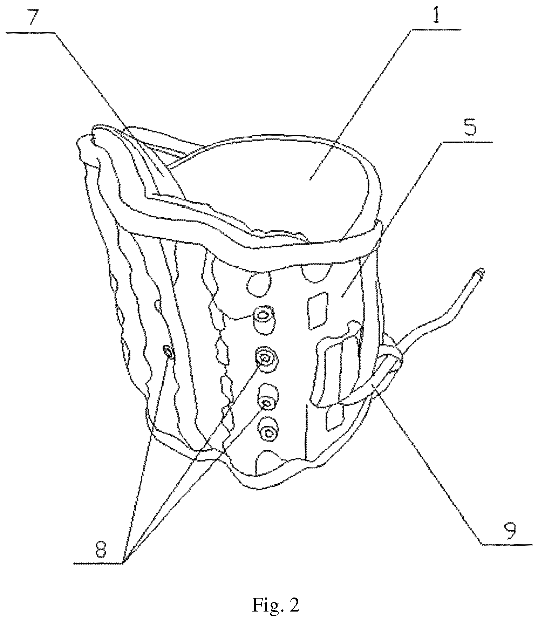

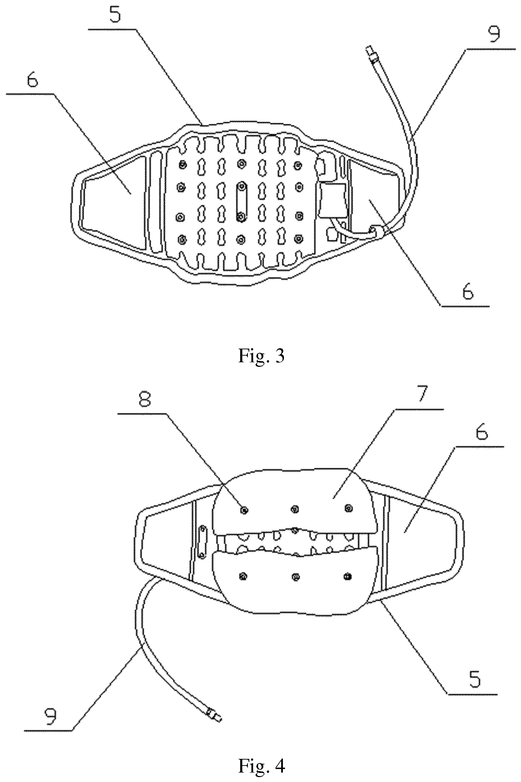

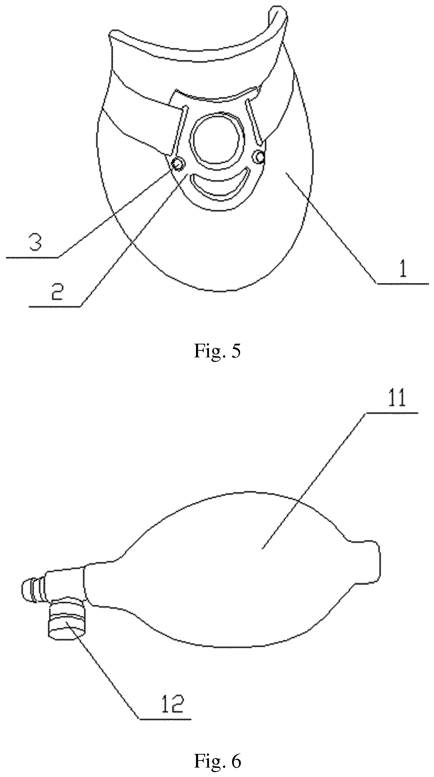

As shown in FIGs. 1-6, the present embodiment adopts a technical solution as follows. It includes a cervical collar 1, a cervical collar support 2, a plastic rivet 3, Velcro hook sides 4, a traction belt 5, Velcro loop sides 6, a first cushion 7, vent holes 8, an inflation tube 9, a connector 10, a hand press ball 11, and an air stopping knob 12. The cervical collar 1 is provided with the cervical collar support 2 at a middle portion at a front end thereof, and the cervical collar support 2 is fastened to the cervical collar 1 through a plastic rivet 3. The Velcro hook sides 4 are attached respectively to left and right ends of the cervical collar support 2. The two Velcro hook sides 4 are fastened respectively to two ends of an outer side of the cervical collar 1. The Velcro loop sides 6 are respectively provided at two ends of an inner side of the traction belt 5. The Velcro loop sides 6 at the two ends of the traction belt 5 are adhered respectively to the Velcro hook sides 4 at the two ends of the outer side of the cervical collar 1. The first cushion 7 is provided at the middle portion of the inner side of the traction belt 5. Multiple vent holes 8 are provided at the middle portion of the traction belt 5. A balloon is provided inside the traction belt 5. One end of the inflation tube 9 is connected to the balloon, and the other end of the inflation tube 9 is provided with the connector 10. The other end of the inflation tube 9 is rotatably connected to one end of the hand press ball 11 through the connector 10. The air stopping knob 12 is provided on the hand press ball 11.

Specifically, the cervical collar 1 is a polymer cervical collar made of a soft spongy material.

### Second Embodiment

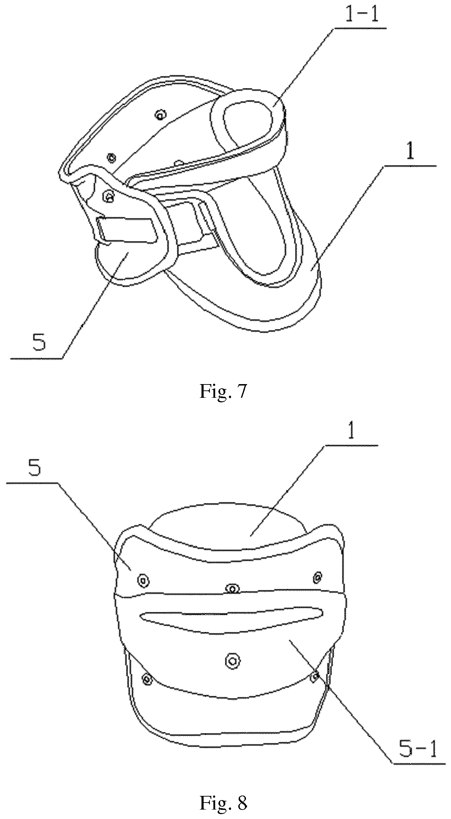

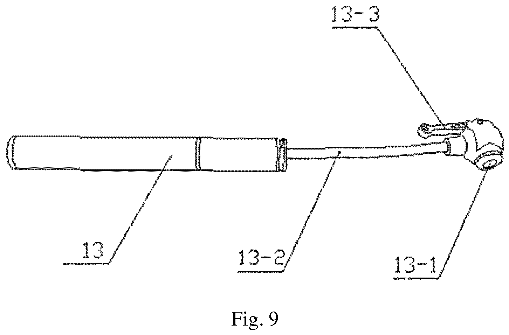

As shown in FIGs. 7-9, this embodiment differs from the first embodiment as follows. The cervical collar 1 is a plastic cervical collar provided with a second cushion 1-1 on its inner side in a region that is to contact the human body. A plastic support 5-1 is provided on the outer side of the traction belt 5 for enhancing the supporting strength of the traction belt. The other end of the inflation tube 9 is connected to a connection port 13-1 in the air pump 13 through the connector 10. An extension tube 13-2 is provided inside the air pump 13. A connector locking means 13-3 is mounted on the lower side of the air pump 13. Other components and connection relationships are the same as those in the first embodiment.

In this embodiment, a separable structure design is adopted in which the cervical collar and the traction belt are assembled through a Velcro before use, so that the cervical vertebra tractor is adjustable to different body sizes of the users. By providing the cervical collar support on the cervical collar, not only can the supporting strength for the cervical vertebra be enhanced, but also the cervical vertebra can be prevented from deformation. By means of the air pump and the hand press ball for inflation, users can choose an inflation means at will according to their usage habits. By virtue of the balloon provided in the traction belt, the traction belt can be inflated into a curved shape which conforms to the physiological curve of the human body, thereby achieving an effective traction.

Basic principles and essential features of the present utility model and advantages of the same have been shown and described above. It should be understood by those skilled in the art that the present utility model is not limited by the above embodiments and that the above embodiments and the specification are intended only to illustrate the principle of the present utility model. Various variations and improvements can be made to the present utility without departing from the spirit and scope of the present utility model. Such variations and improvements fall within the protection scope of the present utility model as defined by the following claims and equivalents thereof.

## Claims

1. A novel cervical vertebra tractor, comprising a cervical collar (1), a cervical collar support (2), a plastic rivet (3), Velcro hook sides (4), a traction belt (5), Velcro loop sides (6), a first cushion (7), vent holes (8), an inflation tube (9), a connector (10), a hand press ball (11), and an air stopping knob (12), in which the cervical collar (1) is provided with the cervical collar support (2) at a middle portion at a front end thereof, the cervical collar support (2) being fastened to the cervical collar (1) through the plastic rivet (3), the Velcro hook sides (4) are attached respectively to left and right ends of the cervical collar support (2), the two Velcro hook sides (4) being fastened respectively to two ends of an outer side of the cervical collar (1), the Velcro loop sides (6) are respectively provided at two ends of an inner side of the traction belt (5), the Velcro loop sides (6) at the two ends of the traction belt (5) are adhered respectively to the Velcro hook sides (4) at the two ends of the outer side of the cervical collar (1), a first cushion (7) is provided at the middle portion of the inner side of the traction belt (5), multiple vent holes (8) are provided at the middle portion of the traction belt (5), a balloon is provided inside the traction belt (5), one end of the inflation tube (9) is connected to the balloon, the other end of the inflation tube (9) is provided to the connector (10) and rotatably connected to one end of the hand press ball (11) through the connector (10), and the air stopping knob (12) is provided on the hand press ball (11).

2. The novel cervical vertebra tractor according to claim 1, wherein the cervical collar (1) is a polymer cervical collar made of a soft spongy material.

3. The novel cervical vertebra tractor according to claim 1, wherein the cervical collar (1) is a plastic cervical collar provided with a second cushion (1-1) on its inner side in a region that is to contact a human body.

4. The novel cervical vertebra tractor according to claim 1, wherein a plastic support (5-1) is provided on an outer side of the traction belt (5).

5. The novel cervical vertebra tractor according to claim 1, wherein the other end of the inflation tube (9) is connected to a connection port (13-1) on an air pump (13) through the connector (10), an extension tube (13-2) is provided inside the air pump (13), and a connector locking means (13-3) is mounted on a lower side of the air pump (13).
